# EUROPÄISCHE PATENTSCHRIFT

(11) **EP 0 340 559 B1**
(45) Veröffentlichungstag und Bekanntmachung des Hinweises auf die Patenterteilung: **14.04.1993**
(21) Anmeldenummer: 89107203.5
(22) Anmeldetag: 21.04.1989
(51) Int. Cl.: C07D 319/06

(54) **Verfahren zur Herstellung von 5-Brom-5-nitro-1,3-dioxan**
Process for the production of 5-bromo-5-nitro-1,3-dioxane
Procédé de préparation de 5-bromo-5-nitro-1,3-dioxane

(30) Priorität: 30.04.1988 DE 3814774
(43) Veröffentlichungstag der Anmeldung: 08.11.1989
(73) Patentinhaber: Henkel Kommanditgesellschaft auf Aktien, 40191 Düsseldorf (DE)
(72) Erfinder: Wüst, Willi, Dr., D-4030 Ratingen (DE); Eskuchen, Rainer, Dr., D-4000 Düsseldorf (DE); Esser, Herbert, D-5210 Troisdorf (DE); Leischner, Hasso, D-4000 Düsseldorf 1 (DE)

(56) Entgegenhaltungen:
- DE-A- 2 913 466
- CHEMICAL ABSTRACTS, Band 105, Nr. 21, 24. November 1986, Columbus, Ohio, USA KUKOVITSKII, D. et al. "5- -Bromo-5-nitro-1+3-dioxanes" Seite 731, Spalte 2, Zusammen-fassung-Nr. 191 101c & SU 1 154 280
- CHEMICAL ABSTRACTS, Band 102, Nr. 12, 25. MUrz 1985, Columbus, Ohio, USA MILSTEIN, ST.R. et al. "Organic synthesis, antibac- terial evaluation, and quantitative structure-activi--ty relationships (QSAR) of cosmetic preservatives relatedto 5-bromo-5-nitro-1,3-dio- -xane. I. Aliphatic analogs" Seite 299, Spalte 2, Zusammen-fassung-Nr. 100 612n & J.Soc.Cosmet.Chem. 1984, 35(2), 73-93

## Beschreibung

Die Erfindung betrifft ein verbessertes Verfahren zur Herstellung des antimikrobiellen Wirkstoffes 5-Brom-5-nitro-1,3-dioxan. Die genannte Verbindung ist aus der DE-B 19 66 920 und aus der DE-C 22 63 206 bekannt. In den genannten Schriften werden auch Herstellverfahren angegeben. So wird nach den Beispielen der DE-B 19 66 920 zum einen mit Benzol oder Methylenchlorid als Lösungsmittel, zum anderen unter Subblimationsbedingungen in einer Subblimationsapparatur gearbeitet. Das deutsche Patent 22 63 206 arbeitet im Lösungsmittel Ethylenchlorid.

Bereits die deutsche Offenlegungsschrift DE 29 13 466 beschreibt ein Verfahren zur Herstellung von 5-Brom-5-nitro-1,3-dioxan durch Umsetzung von 2-Brom-2-nitro-propandiol-1,3 mit Formaldehyd oder Paraformaldehyd in Gegenwart von konzentrierter Schwefelsäure. Es wird jedoch vorgeschlagen, in einem inerten Lösungsmittel zu arbeiten, wobei als Lösungsmittel Tetrachlorethylen verwendet wird. Die Verwendung chlorierter organischer Lösungsmittel wird jedoch für einen technischen Prozeß heute als nachteilig angesehen.

Vor dem Hintergrund dieses Standes der Technik sollte ein Verfahren geschaffen werden, das es erlaubt, 5-Brom-5-nitro-1,3-dioxan ohne Einsatz organischer Lösungsmittel im technischen Maßstab aus rohem, nicht vorgereinigten 2-Brom-2-nitro-1,3-propandiol herzustellen.

Gegenstand der Erfindung ist somit ein Verfahren zur Herstellung von 5-Brom-5-nitro-1,3-dioxan aus 2-Nitro-1,3-propandiol, wobei man in einer ersten Stufe eine Mischung von Brom und wäßriger Bromwasserstofflösung über einen Wärmetauscher im Kreislauf fördert und vor dem Wärmetauscher die wäßrige Lösung eines Alkali-und Erdalkalimetallsalzes des 2-Nitro-1,3-propandiols in einer solchen Geschwindigkeit unter Kühlung zudosiert, daß die maximale Reaktionstemperatur 30 °C nicht übersteigt, und danach in einer zweiten Reaktionsstufe, zu dem 2-Brom-2-nitro-1,3-propandiol, Paraformaldehyd und Schwefelsäure zugibt, oberhalb Raumtemperatur ausreagieren läßt und die organische Phase abtrennt.

### Zur Durchführung der ersten Stufe:

Als Mischung von Brom und Bromwasserstofflösung werden erfindungsgemäß Mischungen von Brom mit einer Bromwasserstofflösung von 50 bis 62 Gew.-% HBr, insbesondere azeotrop siedende wäßrige HBr-Lösung eingesetzt. Das Mischungsverhältnis (molar) HBr : Br₂ beträgt dabei 1 : 3 bis 1 : 10, vorzugsweise 1 : 5 bis 1 : 5,5 und insbesondere um 1 : 5,3.

In diese Mischung werden erfindungsgemäß die Alkalimetallsalze oder auch, soweit wasserlöslich, die Erdalkalimetallsalze des 2-Nitro-1,3-propandiols eingeführt.

Nach einer bevorzugten Ausführungsform des Verfahrens wird das Kaliumsalz des 2-Nitro-1,3-propandiols eingesetzt.

Um die erste Stufe des erfindungsgemäßen Verfahrens mit hohen Raum-Zeit-Ausbeuten durchführen zu können, ist es bevorzugt, die Alkali- und/oder Erdalkalimetallsalze in 40 bis 60 Gew. -%iger wäßriger Lösung oder Suspension einzusetzen.

Erfindungsgemäß wird die beim Eintropfen des Salzes in die Mischung aus Brom und Bromwasserstofflösung entstehende Reaktionswärme über Wärmetauscher abgeführt. Hierbei eignen sich Plattenwärmetauscher oder vorzugsweise Rohrbündelwärmetauscher. Auch anders konstruierte Wärmetauscher sind einsetzbar, wobei generell gilt, daß mit fallender Austauschfläche auch die Eintropfgeschwindigkeit gesenkt werden muß, da bei der Reaktion eine Temperatur von 30 °C vorteilhafterweise nicht überschritten werden sollte.

Nach einer bevorzugten Ausführungsform des Verfahrens der erste Stufe wird die Mischung aus Brom und Bromwasserstofflösung in einem Rührkessel aus korrosionsfestem Werkstoff, beispielsweise Tantal, hergestellt und über einen korrosionsfesten Rohrreaktor mit Hilfe einer Kreiselpumpe in den Rohrbündelwärmetauscher gepumpt und von dort in den Kessel zurückgeleitet. Die Reaktionsmischung wird so lange umgepumpt, bis die gesamte Lösung des Salzes des Nitroalkohols eingeleitet worden ist.

Nach einer bevorzugten Ausführungsform der Erfindung wird, falls am Reaktionsende noch Brom vorhanden ist, was sich an einer Färbung der Lösung zeigt, dieses durch Eingeben eines Reduktionsmittels, beispielsweise von Hydroxylaminlösung, reduziert. Dazu wird die Hydroxylaminlösung nach und nach zugetropft, bis sich der Reaktionsansatz zumindest weitgehend entfärbt hat.

### Zur Durchführung der zweiten Stufe:

Die nach dem erfindungsgemäßen Verfahren erhaltenen Mischungen von Bromiden und 2-Brom-2-nitro-alkoholen können direkt in die zweite Stufe eingesetzt werden. Dazu wird zunächst Paraformaldehyd zugegeben. Das Verhältnis 2-Brom-2-nitro-1,3-propandiol zu Paraformaldehyd beträgt günstigerweise 1 : 1 bis 1 : 1,3, vorzugsweise um 1 : 1,1.

Zur Beschleunigung der Zyklisierungs-Reaktion wird sodann Schwefelsäure zugegeben. Vorzugsweise wird mit 96 bis 98 %iger Schwefelsäure gearbeitet. Das Verhältnis 2-Brom-2₋nitro-1,3-propandiol zu Schwefelsäure soll zwischen 1 : 1 und 1 : 5, vorzugsweise zwischen 1 : 2,5 und 1 : 3,5, liegen.

Dabei hat es sich als günstig erwiesen, den Paraformaldehyd vor der Schwefelsäure in das Reaktionsgemisch einzutragen.

Günstigerweise wird die Zyklisierungs-Reaktion bei Temperaturen oberhalb von Raumtemperatur durchgeführt. Um diese Temperaturen zu erreichen, ist es bevorzugt, die Schwefelsäure so schnell zum Reaktionsansatz zulaufen zu lassen, daß Temperaturen von 70 bis 110 °C, vorzugsweise von 90 bis 100 °C, erreicht werden. Bei diesen Temperaturen wird der Reaktionsansatz wenige Minuten bis zu 2 Stunden belassen, wobei zur Durchmischung gerührt wird. Vorteilhafterweise ist das Reaktionsgefäß mit einem Rückflußkühler ausgerüstet und man läßt den Reaktionsansatz unter Rückfluß sieden. Zum Reaktionsende wird das Rührwerk abgestellt und man trennt die organische Phase, die überwiegend aus 5-Brom-5-nitro-1,3-dioxan besteht, von der Salzlösung ab. Dabei ist es bevorzugt, die Reaktionstemperatur nicht unter 70 °C abzusenken, da bei tieferen Temperaturen die organische Phase zum Kristallisieren neigt.

Der Mechanismus der Reaktion ist zwar nicht bis ins einzelne untersucht, jedoch ist anzunehmen, daß aus den in der Vorstufe entstehenden Salzen (zum Beispiel Kaliumbromid) nach Zugabe der Schwefelsäure Bromwasserstoff entsteht, und daß dieser als starke Säure die Reaktion katalysiert, wobei der zusätzliche Schwefelsäureüberschuß zur Bindung des Reaktionswassers führt. Es hat sich herausgestellt, daß noch höhere Schwefelsäureüberschüsse zu vermeiden sind, da dann unter den Reaktionsbedingungen Oxidationsreaktionen und damit Ausbeuteminderung beobachtet werden.

### Beispiel 1:

### Bromierung des 2-Nitro-1,3-propandiol-Kaliumsalzes

Die Bromierung wurde in einem emaillierten Rührwerksbehälter mit einem Volumen von 1,3 m³ durchgeführt. Dieser war über eine Rohrleitung mit einer Kreiselpumpe mit einem Durchsatz von 10 m³/h, H = 12 m, mit einem 6 qm Rohrbündelwärmetauscher verbunden. Der Auslauf des Wärmetauschers wurde wiederum mit dem Rührwerksbehälter verbunden. Aus einem Brom-Lagerbehälter wurden 228,3 kg Brom und aus einem weiteren Lagerbehälter 35,1 kg 62 %ige HBr in den Rührwerksbehälter gegeben und durch den Produkt-Kühlkreislauf gepumpt. Innerhalb von 1,5 h wurden dann 512,7 kg der Reaktionsmischung aus Beispiel 1 der deutschen Patentanmeldung DE 38 14 772 (Kaliumsalz des 2-Nitro-1,3-propandiols) zudosiert. Die Reaktionstemperatur wurde während der gesamten Reaktionszeit unter 30°C gehalten; bei Erreichen der 30°C wurde der Dosierstrom der Kaliumsalzlösung gedrosselt. Nach beendeter Zugabe und nach einer Nachreaktionszeit von 0,5 h wurde das überschüssige Brom mit 14,8 kg einer 40 Gew.-%igen Hydroxylammoniumchlorid-Lösung innerhalb von 0,25 h reduziert.

### Beispiel 2:

Die Acetalisierung von 2-Brom-2-nitro-propandiol-1,3 wurde in einem 1 ,3 m³ Rührwerksbehälter durchgeführt. Zu den 790,9 kg des bei der Herstellung von 2-Brom-2-nitro-1,3-propandiol anfallenden Reaktionsgemisches nach Beispiel 1 wurden zunächst 42,2 kg Paraformaldehyd gegeben. Hierzu wurden dann 365,9 kg 96 %ige Schwefelsäure so schnell dosiert, daß sich das Reaktionsgemisch auf 90 bis 100 °C erwärmte. Das Reaktionsgemisch wurde nach beendeter Schwefelsäure-Zugabe bei 90 bis 100 °C unter Rückfluß am Sieden gehalten. Nach beendeter Acetalisierung wurde das Reaktionsgemisch auf 70 °C abgekühlt. Dabei bildete sich ein Zwei-Phasen-System aus. In der wässrigen oberen Phase befanden sich die wasserlöslichen Salze, in der unteren organischen Phase das rohe 5-Brom-5-nitro-1,3-dioxan.

Zur Erhöhung der Ausbeute konnten die 953,2 kg der wässrigen oberen Phase mit 521,4 kg 50 %iger Natronlauge auf pH 8 neutralisiert werden, wobei sich als untere Phase eine weitere Menge an 5-Brom-5-nitro-1,3-dioxan bildete.

Zur weiteren Reinigung des rohen 5-Brom-5-nitro-propandiols kann mit 63 kg einer 9 %igen Natriumhydrogencarbonat-Lösung und mit 170 kg Wasser gewaschen werden (bei Temperaturen von 70 °C). Zum Umkristallisieren eignet sich 1,2-Propandiol. Erhalten wurden 247,9 kg reines 5-Brom-5-nitro-1,3-dioxan.

## Patentansprüche

1. Verfahren zur Herstellung von 5-Brom-5-nitro-1,3-dioxan aus 2-Nitro-1,3-propandiol, dadurch gekennzeichnet, daß man in einer ersten Stufe eine Mischung von Brom und wäßriger Bromwasserstofflösung über einen Wärmetauscher im Kreislauf fördert und vor dem Wärmetauscher die wäßrige Lösung eines Alkali- und Erdalkalimetallsaizes des 2-Nitro-1,3-propandiols in einer solchen Geschwindigkeit unter Kühlung zudosiert, daß die maximale Reaktionstemperatur 30 °C nicht übersteigt, und danach in einer zweiten Reaktionsstufe, zu dem 2-Brom-2-nitro-1,3-propandiol, Paraformaldehyd und Schwefelsäure zugibt, oberhalb Raumtemperatur ausreagieren läßt und die organische Phase abtrennt.

2. Verfahren nach Anspruch 1, dadurch gekennzeichnet, daß man das molare Verhältnis von 2-Brom-2-nitro-1,3-propandiol zu Paraformaldehyd (gerechnet als Formaldehyd) auf Werte zwischen 1 : 1 und 1 : 1,3, vorzugsweise um 1 : 1,1, einstellt.

3. Verfahren nach einem der Ansprüche 1 oder 2, dadurch gekennzeichnet, daß man das Verhältnis 2-Brom-2-nitro-1,3-propandiol zu H₂SO₄ zwischen 1 : 1 und 1 : 5, vorzugsweise zwischen 1 : 2,5 und 1 : 3,5, wählt.

4. Verfahren nach einem der Ansprüche 1 bis 3, dadurch gekennzeichnet, daß man zuerst Paraformaldehyd, dann Schwefelsäure zugibt.

5. Verfahren nach einem der Ansprüche 1 bis 4, dadurch gekennzeichnet, daß man die Schwefelsäure in einer solchen Geschwindigkeit zugibt, daß die Temperatur des Reaktionsansatzes auf Werte zwischen 70 und 110 °C, vorzugsweise 90 bis 100 °C, ansteigt.

6. Verfahren nach einem der Ansprüche 1 bis 5, dadurch gekennzeichnet, daß man Stufe 2 oberhalb des Kristallisationspunktes von 5-Brom-5-nitro-1,3-dioxan von etwa 70°C durchführt und oberhalb dieser Temperatur die organische Phase von der Salzlösung abtrennt.

## Claims

1. A process for the production of 5-bromo-5-nitro-1,3-dioxane from 2-nitropropane-1,3-diol, characterized in that, in a first step, a mixture of bromine and aqueous hydrogen bromide solution is circulated through a heat exchanger and, before the heat exchanger, an aqueous solution of an alkali metal and alkaline earth metal salt of 2-nitropropane-1,3-diol is added with cooling at such a rate that the maximum reaction temperature does not exceed 30°C and, in a second reaction step, paraformaldehyde and sulfuric acid are added to the 2-bromo-2-nitropropane-1,3-diol, the mixture is left to react out above room temperature and the organic phase is separated off.

2. A process as claimed in claim 1, characterized in that the molar ratio of 2-bromo-2-nitropropane-1,3-diol to paraformaldehyde (expressed as formaldehyde) is adjusted to values of 1:1 to 1:1.3 and preferably to values of around 1:1.1.

3. A process as claimed in claim 1 or 2, characterized in that the ratio of 2-bromo-2-nitropropane-1,3-diol to H₂SO₄ is between 1:1 and 1:5 and preferably to between 1:1.5 and 1:3.5.

4. A process as claimed in any of claims 1 to 3, characterized in that first paraformaldehyde and then sulfuric acid is added.

5. A process as claimed in any of claims 1 to 4, characterized in that the sulfuric acid is added at such a rate that the temperature of the reaction mixture rises to values of 70 to 110°C and preferably to values of 90 to 100°C.

6. A process as claimed in any of claims 1 to 5, characterized in that step 2 is carried out above the crystallization point of 5-bromo-5-nitro-1,3-dioxane of approximately 70°C and the organic phase is separated from the salt solution above that temperature.

## Revendications

1. Procédé de préparation de 5-bromo-5-nitro-1,3-dioxane à partir de 2-nitrol-1,3-propanediol, caractérisé en ce qu'on fait circuler dans une première étape un mélange de brome et de solution aqueuse de bromure d'hydrogène par passage dans un échangeur thermique et qu'on ajoute avant l'échangeur thermique, la solution aqueuse d'un sel de métal alcalin ou alcalino-terreux du 2-nitro-1,3-propanediol en refroidissant, à une vitesse telle que la température maximale ne dépasse pas 30°C, et ensuite dans une deuxième étape de réaction on ajoute du paraformaldéhyde et de l'acide sulfurique au 2-bromo-2-nitro-1,3-propanediol, et on fait réagir à une température supérieure à la température ambiante et qu'on sépare la phase organique.

2. Procédé selon la revendication 1, caractérisé en ce qu'on ajuste la proportion molaire du 2-bromo-2-nitro-1,3-propanediol au paraformaldéhyde (calculé sous forme de formaldéhyde) à des valeurs comprises entre 1:1 et 1:1,3, de préférence autour de 1:1,1.

3. Procédé selon l'une des revendications 1 ou 2, caractérisé en ce qu'on choisit la proportion du 2-bromo-2-nitro-1,3-propanediol à H₂SO₄ entre 1:1 et 1:5, de préférence entre 1:2,5 et 1:3,5.

4. Procédé selon l'une des revendications 1 à 3, caractérisé en ce qu'on ajoute d'abord le paraformaldéhyde, puis l'acide sulfurique.

5. Procédé selon l'une des revendications 1 à 4, caractérisé en ce qu'on ajoute l'acide sulfurique à une vitesse telle que la température du mélange réactionnel monte à des valeurs comprises entre 70 et 110°C, de préférence de 90 à 100°C.

6. Procédé selon l'une des revendications 1 à 5, caractérisé en ce qu'on réalise l'étape 2 au-dessus du point de cristallisation du 5-bromo-5-nitro-1,3-dioxane d'environ 70°C et qu'on sépare la phase organique de la solution saline au-dessus de cette température.
